# EUROPEAN PATENT SPECIFICATION

(11) **EP 3 058 094 B1**
(45) Date of publication and mention of the grant of the patent: **17.01.2018**
(21) Application number: 14810038.1
(22) Date of filing: 15.10.2014
(51) Int. Cl.: C12Q 1/68

(54) **METHOD FOR DETECTION OF DECREASED SUSCEPTIBILITY FOR ANTICANCER ADJUVANT CHEMOTHERAPY IN BREAST CANCER PATIENTS**
VERFAHREN ZUM NACHWEIS VON VERRINGERTER SUSZEPTIBILITÄT FÜR ADJUVANTE CHEMOTHERAPIE BEI BRUSTKREBSPATIENTEN
MÉTHODE DE DÉTECTION D'UNE BAISSE DE SENSIBILITÉ VIS-À-VIS D'UNE CHIMIOTHÉRAPIE ADJUVANTE ANTICANCÉREUSE CHEZ DES PATIENTES ATTEINTES D'UN CANCER DU SEIN

(30) Priority: 15.10.2013 PL 40565613
(43) Date of publication of application: 24.08.2016
(73) Proprietor: Wroclawskie Centrum Badan EIT + Sp. z o.o., 54-066 Wroclaw (PL)
(72) Inventor: PULA, Bartosz, 52-210 Wroclaw (PL); OLBROMSKI, Mateusz, 98-400 Wieruszów (PL); AMBICKA, Aleksandra, 44-120 Pyskowice (PL); GOMULKIEWICZ, Agnieszka, 58-200 Dzierzoniów (PL); RYS, Janusz, 30-611 Kraków (PL); PODHORSKA-OKOLÓW, Marzena, 53-303 Wroclaw (PL); SIEW, Hwa, Ong, 02-12. Singapore 117576 (SG); DZIEGIEL, Piotr, 51-650 Wroclaw (PL)
(74) Representative: Witek, Rafal
(86) International application number: PCT/PL2014/000112
(87) International publication number: WO 2015/057090

(56) References cited:
- WO-A1-2011/109637
- WO-A1-2013/014296
- A. GOLDHIRSCH ET AL: "Strategies for subtypes--dealing with the diversity of breast cancer: highlights of the St Gallen International Expert Consensus on the Primary Therapy of Early Breast Cancer 2011", ANNALS OF ONCOLOGY, vol. 22, no. 8, 27 June 2011 (2011-06-27), pages 1736-1747, XP055164503, ISSN: 0923-7534, DOI: 10.1093/annonc/mdr304 cited in the application
- SILVIA DARB-ESFAHANI ET AL: "Gross cystic disease fluid protein 15 (GCDFP-15) expression in breast cancer subtypes", BMC CANCER, BIOMED CENTRAL, LONDON, GB, vol. 14, no. 1, 28 July 2014 (2014-07-28), page 546, XP021191720, ISSN: 1471-2407, DOI: 10.1186/1471-2407-14-546
- ALI NADERI ET AL: "R Prolactin-induced protein mediates cell invasion and regulates integrin signaling in estrogen receptor-negative breast cancer", BREAST CANCER RESEARCH, vol. 14, 31 December 2012 (2012-12-31), page r111, XP055164466,
- BOUTTEVILLE C ET AL: "[Hormonal regulation of GCDFP-15 secretion in breast tumors. Study of cultured cells].", BULLETIN DU CANCER 1989, vol. 76, no. 8, 1989, pages 805-811, XP8174529, ISSN: 0007-4551

## Description

The object of the invention is a method for detection of decreased susceptibility for anticancer adjuvant chemotherapy in breast cancer patients. The invention provides prediction test that enables both rapid classification of patients in terms of their response for adjuvant chemotherapy, as well as undertaking appropriate therapeutic intervention.

Breast cancer is one of the most commonly diagnosed malignant tumours in women. In Europe, there are reported approximately 400.000 new cases and about 132. 000 deaths resulting from this malignant cancer, annually. Likewise, comparable (per habitants) number of diagnosed breast cancer cases reaching 180 000 and mortality of about 40 000 cases annually is observed in United States. In Poland, there are 16 000 new incidences diagnosed every year. Moreover, breast cancer is the most common cause of death in the group of women aged 20-59 years [1]. Breast cancers are characterised by high heterogeneity, however in clinical practise they are routinely classified in non-histological manner (in order to undertake an appropriate therapy) based on the expression of the following markers: estrogen receptor α (ERα), progesterone receptor (PR), human epidermal growth factor receptor 2 (HER2) and Ki-67 antigen expression [2, 3]. The majority of breast cancers (70%) show ERα and PR (ERα+/PR+) expression, 20% are characterised by HER2 (HER2+++) overexpression and in approximately 10% of cases above-mentioned markers are not expressed (so-called triple negative breast cancers, TNBC). Those proteins are the main prediction factors for the use of anti-estrogenic (ERα+ tumours) and trastuzumab (HER2+++ tumours) therapy.

Prolactin-inducible protein (PIP), also called serum actinin-binding protein (SABP) or GCDFP-15 (gross cystic disease fluid protein-15) is a 15 kDa glycoprotein that is found in both normal and cancer cells, especially those showing characteristics of apocrine differentiation [4-6]. PIP expression was shown also in primary tumours and breast cancer metastasis (14% to 74% cases, depending on the type of lesion) [5, 7-10]. Among invasive ductal breast cancers (IDC) subtypes studied, the lowest PIP expression was shown in so-called TNBC cases, which are characterised by the least favourable clinical course [10]. Moreover, it was shown that the incubation of human breast cancer cell lines with PIP leads to the increase of their proliferative potential, irrespectively of pro-proliferative action of estrogen [11-13]. Additionally, inhibition of PIP expression in ERα+ and ERα- lines leads to the decrease of their invasiveness *in vitro.* [14,15]

The publication by Fisher *et al.* is known in the art, where the studies on *PIP* mRNA expression level are described, as one of 7 genes studied (*mam, mamb, PIP, CK19, mucl, PSE, CEA*) in lymph nodes in which no metastatic lesions were shown [16]. However, this study was performed on clinical material from lymph nodes, but not from primary IDC tumours. Moreover, the aim of the study was: (1) to verify the usefulness of the expression of the above-mentioned panel of genes (also *PIP*) for the detection of micro-metastasis in lymph nodes, and (2) to determine the prognostic significance (overall survival) of the expression of the genes studied [16]. WO2013/014296 (Sividon Diagnostics GmbH) describes the use of expression marker sets including PIP to predict response to breast cancer treatment. There are a number of publications, wherein the application of immunohistochemical method for the evaluation of PIP expression in the characterisation of biology of breast cancers is described. In the paper by Chia *et al.,* the usefulness of PIP protein expression for diagnostics of recurrent tumours of breast cancers was studied [17]. On the other hand, Lewis *et al.* studied the expression of PIP protein in various subtypes of ductal breast cancers [18]. Luo *et al.* also determined the expression of PIP with the use of immunohistochemical technique as well as tissue microarrays (TMA) in 833 cases of IDC and they evaluated the relationship in accordance with clinical pathological data from patients [19]. The authors have shown that low PIP expression was related with advanced age of patients, lower degree of tumour malignancy and the lack of presence of metastasis to lymph nodes. In this study, despite of the analysis of the relationship of PIP expression and clinical pathological data, no prognostic or predictive value for this protein was determined [19]. Moreover, in any of the above-mentioned publications no *PIP* mRNA expression was determined with the use of real-time PCR technique.

In the paper of Fritzsche *et al.* also studied was the usefulness of immunohistochemical method for the evaluation of PIP expression in the group of 165 patients with breast cancer. In the studied group of patients, PIP expression was determined immunohistochemically and it was associated with longer survival in patients. However, only 28 patients received unspecified adjuvant chemotherapy. Therefore, based on the disclosed results, it is not possible to determine the usefulness of PIP as the predictive marker [9].

The level of *PIP* mRNA expression was studied also by Pagani *et al.* [20]. In this experiment, the expression of *PIP* mRNA was studied in ductal breast cancers with the use of *in situ* hybridisation method (26 cases) and Northern-blot technique (17 cases). Additionally, PIP expression was evaluated also with the use of immunohistochemical technique in 33 cases of breast cancers. High PIP expression in breast cancer cells, both at the mRNA (*in situ* hybridisation) and protein level (immunohistochemical method) was associated with longer, recurrence-free survival in patients [20]. However, the method used in the above-mentioned study differs from the real-time PCR technique, as *in situ* hybridisation method does not enable to determine the level of PIP transcript (mRNA), but allows to show its presence only. Moreover, in that publication no issue was discussed regarding the chemoresistance of studied breast cancers - thus, it is impossible to evaluate this parameter based on the studies described above [20]. In the above-mentioned publication, high PIP expression on the protein level, as measured by immunohistochemical method, was associated also with longer, recurrence-free survival in patients.

The results of the recent studies enabled to increase the efficacy of breast cancer treatments, however they remain unsatisfactory. Moreover, there are still no inexpensive and fully effective tests enabling reliable determination of clinical course of the disease.

Currently available commercial tests are based on gene signatures. They include *inter alia* Oncotype DX™ (21 genes) and MammaPrint™ (70 genes), which can be of help to medical personnel in planning breast cancer chemotherapy and evaluation of risk of cancer cells spreading. However, they have not been shown to be more effective in comparison to previously employed determination of status of ERα, PR and HER2 receptors. Additionally, the determination of expression of potential markers not only in cancer cells, but also in other cells being the elements of tumour (e.g. fibroblasts, endothelial cells, macrophages etc.) is a significant drawback of those tests. Furthermore, the costs of above-mentioned multigenic tests are relatively high, which is the reason why they have not found wider application in everyday clinical practise. Previous studies have shown that PIP expression was present in normal acinar breast cells as well as in cancer cells. No presence of PIP expression was confirmed in other types of the above-mentioned cells being parts of breast cancer.

The object of the invention is a method for detection of decreased susceptibility for anticancer adjuvant chemotherapy with cytostatic from the group of oxazaphosphorines and antibiotic from the group of anthracyclines, in breast cancer patients. The method is characterised in that the evaluation of *PIP* gene expression is performed in the sample of tissue material from primary tumour of ductal breast cancer in the isolated total RNA with the use of real-time PCR method, wherein the statistically significant lower expression of *PIP* mRNA indicates decreased susceptibility for anticancer adjuvant chemotherapy with cytostatic from the group of oxazaphosphorines and antibiotic from the group of anthracyclines.
Preferably, the breast cancer is invasive ductal breast cancer (IDC).
Preferably, cytostatic from the group of oxazaphosphorines is cyclophosphamide (C).
Preferably, antibiotic from the group of anthracyclines is doxorubicin (A).
Preferably, adjuvant chemotherapy includes 4xAC or 6xAC cycle.
Preferably, a value with p < 0.05 is considered statistically significant lower.
Preferably, the level of *PIP* mRNA expression is evaluated in frozen material from primary tumour.

The object of the invention is the development and the application of diagnostic test enabling the determination of the responsiveness of patient with invasive ductal breast cancer (IDC) for the cycle of chemotherapy 4xAC (4 courses of doxorubicin [60 mg/m² of body surface] and [600 mg/m² of body surface] cyclophosphamide) and 6xAC. The test is performed *in vitro* and is characterised by the determination of *PIP* gene expression in isolated total RNA from the material of primary IDC with the use of real-time PCR method. The evaluation of *PIP* mRNA expression level enables to classify patients to the group of cases which will positively respond to the treatment (responder; R) or to the group, in which there will be no remission or in which there will be recurrence in the next 60 months period (non-responder; N).

The method of the present invention is much more sensitive in comparison to both of the above described methods used by Pagani *et al.* [20] as well as to immunohistochemical method used in the most of the published papers.

The test is expected to find an application in two clinical situations: 1) before the onset of chemotherapy course and/or 2) after completion (or during) of 4xAC or 6xAC chemotherapy. In both cases, frozen tumour fragments will be employed for the examinations (taken during routine mammectomy, quadrantectomy or core needle biopsy procedure) before the onset of chemotherapy, with the objective to determine expression of *PIP* mRNA. In case of low *PIP* mRNA expression in the analysed sample of breast cancer - in the first case (1) it is recommended to administer another treatment scheme based on different chemotherapeutics, wherein in the other case (2), when patient has completed (or is under) chemotherapy 4xAC or 6xAC, it is recommended to administer another combination of chemotherapeutics and in the case of patient being under the course of 4xAC or 6xAC - to terminate it and to implement another chemotherapy scheme.

The application of the invention and the classification of patient with its use to N group will enable employment of extended therapeutic management is case of the treatment with 4xAC or 6xAC adjuvant chemotherapy course. An advantageous effect is direct minimisation of adverse side effects (cardio-, nephro-, mielo- and neurotoxicity) of applied anticancer therapy, as well as indirect reduction of overall treatment cost.

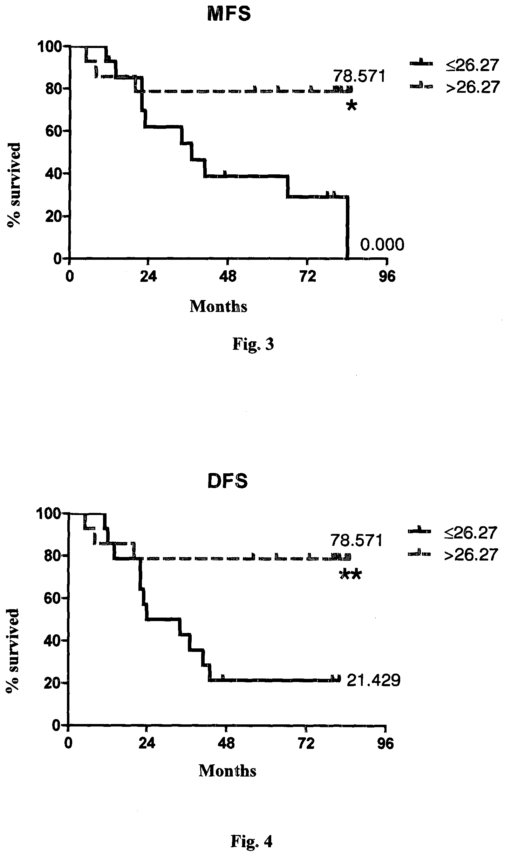

The object of the invention is shown in the example (Figure 1), wherein the degree of *PIP* mRNA expression intensity was shown in analysed IDC cases, depending on the expression of estrogen receptors (ER); *p<0.05; Mann-Whitney test. Figure 2 shows the level of *PIP* mRNA expression intensity in analysed cases of triple negative IDC (TNBC) and in other histological types of IDC; *p<0.05; Mann-Whitney test. Figure 3 shows metastasis free survival (MFS) curves depending on the level of *PIP* mRNA expression; *p<0.05; Mantel-Cox test. Figure 4 shows disease free survival (DFS) curves depending on the level of *PIP* mRNA expression; *p<0.05; Mantel-Cox test.

### Example

The object of the invention is shown on a group of 28 patients with IDC, among which 14 were diagnosed with cancer recurrence within five years from the onset of the treatment (non-responder; N) and 14 patients with no recurrence of the disease observed within above-mentioned time period (responder; R). All patients included in the treatment received course of 4xAC or 6xAC chemotherapy, while anti-estrogenic treatment was employed in all patients whose tumours were characterised by ERα expression (ER+). Summary of clinical pathological data from study group is shown in Table I.

**Table I. Summary of clinical pathological data from IDC patients.**

| **Parameter** | **N** | **%** |
|---|---|---|
| **Age** | | |
| ≤ 55 | 12 | 42.9 |
| > 55 | 16 | 57.1 |
| **pT** | | |
| pT1 | 4 | 14.3 |
| pT2, pT3, pT4 | 24 | 85.7 |
| **pN** | | |
| pN0 | 10 | 35.7 |
| pN1, pN2, pN3 | 18 | 64.3 |
| **ER** | | |
| Positive (ER+) | 10 | 35.7 |
| Negative (ER-) | 18 | 64.3 |
| **PR** | | |
| Positive (PR+) | 10 | 35.7 |
| Negative (ER-) | 18 | 64.3 |
| **HER2** | | |
| Positive (HER2+) | 7 | 25.0 |
| Negative (HER2-) | 21 | 75.0 |
| **Triple-negative** | | |
| TNBC | 11 | 39.3 |
| Other types | 17 | 60.7 |

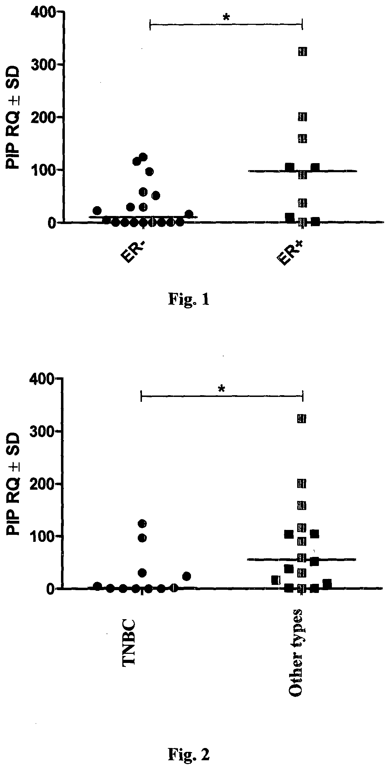

It was shown that *PIP* mRNA expression was statistically significant higher in ER+ tumours in comparison to ER- tumours (p<0.05, Mann-Whitney test; Figure 1). Moreover, statistically significant lower *PIP* mRNA expression was reported in TNBC tumours in comparison to other histological subtypes of IDC (p<0.05, Mann-Whitney test; Figure 2). Statistical analysis did not reveal correlation between *PIP* mRNA expression and the expression of PR and HER2 receptors. Based on median value of *PIP* mRNA expression in whole study group, it was divided into 2 subgroups subjected to statistical analysis in regard to survival in patients. Mantela-Cox test have shown statistically significant shorter MFS (metastasis free survival; p<0.05) and DFS (disease - free survival; p<0.005) in patients whose tumours were characterised by low *PIP* mRNA expression. Low *PIP* mRNA expression (lower than *PIP* mRNA expression median of study IDC cases) in a group of patients treated with 4xAC or 6xAC chemotherapy was correlated with weak response for treatment and increased risk of recurrence in 48-months period from the moment of application of anti-cancer treatment. During the above-mentioned observation period in the group of patients with low level of *PIP* mRNA expression, local recurrence in 11/14 (78.6%) of patients and disease process progression as new tumour occurrence outside of breast in 10/14 of patients (71.4%) was reported. In the case of patients in a group of high *PIP* mRNA expression, corresponding portion of cancerous process recurrence was 3/14 (21.4%, local recurrence) and 3/14 (21.4%, new tumour, respectively.

***Total RNA isolation.*** Total RNA from tumours of breast cancer was isolated with the use of isolation kit - RNeasy Mini Kit (Qiagen, USA), according to protocol. Approximately 30mg of sample was placed in 600µl RLT buffer with addition of 1µl β-mercaptoethanol. Tissues were homogenised with QIAschredder homogeniser (Qiagen, Germany). Centrifugation was performed for 3 min at 13 400 rpm. Supernatant was transferred into new Eppendorf-type tubes. One-volume of 70% ethanol was added. The samples were mixed thoroughly. The solution was transferred in 700 µl portions into columns packed with resin for nucleic acids purification, placed in 2ml tubes. Centrifugation was performed for 15 s at 10 000 rpm. 700µl of RW1 buffer was placed into columns. Centrifugation was performed for 15 s at 10000 rpm. 500µl of RPE buffer was placed into columns. Centrifugation was performed for 15 s at 10000 rpm. Columns were placed in new 2ml tubes. Centrifugation was performed for 1 min at 13 400 rpm. 30µl RNases-free water was placed [into columns] to elute RNA and it was incubated for 4 min. Centrifugation was performed for 1 min at 10 000 rpm.

***Reverse transcription reaction.*** Reverse transcription reaction was performed on the template of isolated total RNA with the use of QuantiTect® Reverse Transcription Kit (Qiagen, Germany). The composition or reaction mixture having final volume of 20µl is shown in Table II. The whole was mixed and placed in DNA Engine® Peltier Thermal Cycler (Bio-Rad, USA). Reverse transcription reaction was conducted for 45 min at temperature of 42°C. Next, reverse transcription reaction was thermally inactivated by heating samples for 5 min at temperature of 99°C. Obtained cDNA was stored at temperature of -20°C until PCR reaction was performed.

**Table II. The volume of reaction mixture for reverse transcription reaction.**

| Component | Volume |
|---|---|
| **RNA template (400ng)** | **10µl** |
| **Reaction buffer (10x concentrated)** | **2µl** |
| **nucleotides mix, dNTP (5mM)** | **0.8µl** |
| **primers oligo-dt (10µM)** | **2µl** |
| **RNases inhibitor (10U/µl)** | **1µl** |
| **reverse transcriptase** | **1µl** |
| **RNases-free water** | **3,2µl** |

***Real time PCR reaction.*** For real-time PCR reaction TaqMan primers from Applied Biosystems were used, which are specific for reference gene *SDHA* (Hs00188166_m1) and for study gene PIP (Hs01114172_ml). cDNA was amplified in 1x Universalm Master Mix buffer (Applied Biosystems, USA) with the use of 7500 Real-Time PCR System apparatus from Applied Biosystems company. Amplification was conducted in following conditions: 45 cycles: 95°C for 15 seconds, 60°C for 1 minute preceded by 95°C for 10 minutes and 50°C for 2 minutes. Real-time PCR reaction was conducted in triplicates. Analysis of expression was performed with the use of ΔΔCt method.

***Statistical analysis.*** Obtained results were subjected to statistical analysis with the use of Prism 5.0 programme (GraphPad, CA, USA). Mann-Whitney test was used for the comparison of two groups of nonparametric variables, while Mantel-Cox test was employed for the analysis of disease free survival (DFS) as well as metastasis free survival (MFS). For p<0.05 the difference was statistically significant.

### Literature

1. Siegel, R., D. Naishadham, and A. Jemal, Cancer statistics, 2013. CA Cancer J Clin, 2013. 63(1): p. 11-30.
2. Goldhirsch, A., et al., Thresholds for therapies: highlights of the St Gallen International Expert Consensus on the primary therapy of early breast cancer 2009. Ann Oncol, 2009. 20(8): p. 1319-29.
3. Goldhirsch, A., et al., Strategies for subtypes--dealing with the diversity of breast cancer: highlights of the St. Gallen International Expert Consensus on the Primary Therapy of Early Breast Cancer 2011. Ann Oncol, 2011. 22(8): p. 1736-47.
4. Caputo, E., et al., Structural study of GCDFP-15/gp17 in disease versus physiological conditions using a proteomic approach. Biochemistry, 2003. 42(20): p. 6169-78.
5. Mazoujian, G., et al., Expression of GCDFP-15 in breast carcinomas. Relationship to pathologic and clinical factors. Cancer, 1989. 63(11): p. 2156-61.
6. Mazoujian, G., et al., Immunohistochemistry of a gross cystic disease fluid protein (GCDFP-15) of the breast. A marker of apocrine epithelium and breast carcinomas with apocrine features. Am J Pathol, 1983. 110(2): p. 105-12.
7. Wick, M.R., et al., Gross cystic disease fluid protein-15 as a marker for breast cancer: immunohistochemical analysis of 690 human neoplasms and comparison with alpha-lactalbumin. Hum Pathol, 1989. 20(3): p. 281-7.
8. Bhargava, R., S. Beriwal, and D.J. Dabbs, Mammaglobin vs GCDFP-15: an immunohistologic validation survey for sensitivity and specificity. Am J Clin Pathol, 2007. 127(1): p. 103-13.
9. Fritzsche, F.R., et al., Co-expression and prognostic value of gross cystic disease fluid protein 15 and mammaglobin in primary breast cancer. Histol Histopathol, 2007. 22(11): p. 1221-30.
10. Huo, L., et al., Gross cystic disease fluid protein-15 and mammaglobin A expression determined by immunohistochemistry is of limited utility in triple-negative breast cancer. Histopathology, 2013. 62(2): p. 267-74.
11. Blais, Y., et al., Interleukin-4 and interleukin-13 inhibit estrogen-induced breast cancer cell proliferation and stimulate GCDFP-15 expression in human breast cancer cells. Mol Cell Endocrinol, 1996. 121(1): p. 11-8.
12. Cassoni, P., et al., Mitogenic effect of the 15-kDa gross cystic disease fluid protein (GCDFP-15) on breast-cancer cell lines and on immortal mammary cells. Int J Cancer, 1995. 60(2): p. 216-20.
13. Baniwal, S.K., et al., Prolactin-Induced Protein (PIP) Regulates Proliferation of Luminal A Type Breast Cancer Cells in an Estrogen-Independent Manner. PLoS One, 2013. 8(6): p. e62361.
14. Naderi, A. and M. Meyer, Prolactin-induced protein mediates cell invasion and regulates integrin signaling in estrogen receptor-negative breast cancer. Breast Cancer Res, 2012. 14(4): p. R111.
15. Baniwal, S.K., et al., Runx2 controls a feed-forward loop between androgen and prolactin-induced protein (PIP) in stimulating T47D cell proliferation. J Cell Physiol, 2012. 227(5): p. 2276-82.
16. Fisher, C.S., et al., Molecular detection of micrometastatic breast cancer in histopathology-negative axillary lymph nodes fails to predict breast cancer recurrence: a final analysis of a prospective multi-institutional cohort study. Ann Surg Oncol, 2010. 17 Suppl 3: p. 312-20.
17. Chia, S.Y., et al., Utility of mammaglobin and gross cystic disease fluid protein-15 (GCDFP-15) in confirming a breast origin for recurrent tumors. Breast, 2010. 19(5): p. 355-9.
18. Lewis, G.H., et al., Relationship between molecular subtype of invasive breast carcinoma and expression of gross cystic disease fluid protein 15 and mammaglobin. Am J Clin Pathol, 2011. 135(4): p. 587-91.
19. Luo, M.H., et al., Expression of mammaglobin and gross cystic disease fluid protein-15 in breast carcinomas. Hum Pathol, 2013. 44(7): p. 1241-50.
20. Pagani, A., et al., PIP/GCDFP-15 gene expression and apocrine differentiation in carcinomas of the breast. Virchows Arch, 1994. 425(5): p. 459-65.

## Claims

1. The method for detection of decreased susceptibility for anticancer adjuvant chemotherapy with cytostatic from the group of oxazaphosphorines and antibiotic from the group of anthracyclines in breast cancer patients, **characterized in that** the evaluation of *PIP* gene expression is performed in the sample of tissue material taken from patient from primary tumour of invasive ductal breast cancer (IDC) in the isolated total RNA with the use of real-time PCR method, wherein the statistically significant lower expression of *PIP* mRNA indicates decreased susceptibility for anticancer adjuvant chemotherapy with cytostatic from the group of oxazaphosphorines and antibiotic from the group of anthracyclines.

2. A method according to claim 1, **characterized in that** cytostatic from the group of oxazaphosphorines is cyclophosphamide (C).

3. A method according to claim 1, **characterized in that** antibiotic from the group of anthracyclines is doxorubicin (A).

4. A method according to claim 1, **characterized in that** adjuvant chemotherapy includes 4xAC or 6xAC cycle.

5. A method according to claim 1, **characterized in that** statistically significant lower is considered value with p < 0.05.

6. A method according to claim 1, **characterized in that** the level of *PIP* mRNA expression is evaluated in frozen material from primary tumour.

## Patentansprüche

1. Ein Verfahren zur Detektion verminderter Empfindlichkeit für adjuvante Antikrebschemotherapie mit einem Zytostatikum aus der Gruppe der Oxazaphosphorine und einem Antibiotikum aus der Gruppe der Anthracycline in Brustkrebspatienten, dadurch charakterisiert, dass die Bestimmung der PIP-Genexpression in einer dem Patienten entnommenen Gewebematerialprobe aus dem Primärtumor invasiven duktalen Brustkrebses (IDC) in der isolierten Gesamt-RNA unter Verwendung des Echtzeit-PCR-Verfahrens durchgeführt wird, wobei statistisch signifikant niedrigere Expression von PIP-mRNA verminderte Empfindlichkeit für adjuvante Antikrebschemotherapie mit einem Zytostatikum aus der Gruppe der Oxazaphosphorine und einem Antibiotikum aus der Gruppe der Anthracycline anzeigt.

2. Ein Verfahren nach Anspruch 1, dadurch charakterisiert, dass das Zytostatikum aus der Gruppe der Oxazaphosphorine Cyclophosphamid (C) ist.

3. Ein Verfahren nach Anspruch 1, dadurch charakterisiert, dass das Antibiotikum aus der Gruppe der Anthracycline Doxorubicin (A) ist.

4. Ein Verfahren nach Anspruch 1, dadurch charakterisiert, dass die adjuvante Antikrebschemotherapie 4xAC- oder 6xAC-Zyklen umfasst.

5. Ein Verfahren nach Anspruch 1, dadurch charakterisiert, dass als statistisch signifikant ein Wert p < 0.05 angesehen wird.

6. Ein Verfahren nach Anspruch 1, dadurch charakterisiert, dass die Höhe der PIP-mRNA-Expression in gefrorenem Material aus Primärtumor bestimmt wird.

## Revendications

1. Procédé de détection d'une baisse de sensibilité à une chimiothérapie adjuvante anticancéreuse à l'aide d'un cytostatique du groupe des oxazaphosphorines et d'un antibiotique du groupe des anthracyclines chez des patientes atteintes d'un cancer du sein, **caractérisé en ce que** l'évaluation de l'expression du gène *PIP* est réalisée dans l'échantillon de matériel tissulaire prélevé chez la patiente à partir d'une tumeur primaire d'un cancer canalaire invasif du sein (IDC, Invasive Ductal breast Cancer) dans l'ARN total isolé à l'aide d'un procédé PCR en temps réel, dans lequel une expression plus faible statistiquement significative de l'ARN de *PIP* indique une baisse de la sensibilité à une chimiothérapie adjuvante anticancéreuse à l'aide d'un cytostatique du groupe des oxazaphosphorines et d'un antibiotique du groupe des anthracyclines.

2. Procédé selon la revendication 1, **caractérisé en ce que** le cytostatique du groupe des oxazaphosphorines est le cyclophosphamide (C).

3. Procédé selon la revendication 1, **caractérisé en ce que** l'antibiotique du groupe des anthracyclines est la doxorubicine (A).

4. Procédé selon la revendication 1, **caractérisé en ce que** la chimiothérapie adjuvante inclut le cycle 4xAC ou 6xAC.

5. Procédé selon la revendication 1, **caractérisé en ce qu'**une valeur plus faible statistiquement significative est une valeur ayant un p < 0,05.

6. Procédé selon la revendication 1, **caractérisé en ce que** le niveau d'expression de l'ARNm de *PIP* est évalué dans du matériel congelé provenant d'une tumeur primaire.
